# EUROPÄISCHE PATENTSCHRIFT

(11) **EP 0 907 888 B1**
(45) Veröffentlichungstag und Bekanntmachung des Hinweises auf die Patenterteilung: **26.06.2002**
(21) Anmeldenummer: 97916413.4
(22) Anmeldetag: 29.03.1997
(51) Int. Cl.: G01N 33/533, G01N 33/535, G01N 33/543

(54) **VERFAHREN ZUR MARKIERUNG VON MOLEKÜLEN**
MOLECULE-MARKING METHOD
PROCEDE DE MARQUAGE DE MOLECULES

(30) Priorität: 02.04.1996 DE 19612650
(43) Veröffentlichungstag der Anmeldung: 14.04.1999
(73) Patentinhaber: Evotec OAI AG, 22525 Hamburg (DE)
(72) Erfinder: SCHIERHOLZ, Bernd, D-22765 Hamburg (DE); BAUER, Günter, J., D-24568 Kaltenkirchen (DE); WERNER, Irmgard, D-21629 Neu Wulmstorf (DE); MEYER-ALMES, Franz-Josef, D-58636 Iserlohn (DE); KREUZER, Olivier, D-10738 Berlin (DE)
(74) Vertreter: Meyers, Hans-Wilhelm, Dr.Dipl.-Chem.
(86) Internationale Anmeldenummer: EP9701600
(87) Internationale Veröffentlichungsnummer: WO9737221

(56) Entgegenhaltungen:
- DE-A- 3 912 046
- US-A- 4 874 813
- US-A- 5 244 816
- US-A- 5 290 925

## Beschreibung

Die vorliegende Erfindung betrifft ein Verfahren zur Markierung von Molekülen gemäß Anspruch 1 sowie eine Vorrichtung zur Durchführung des Verfahrens gemäß Anspruch 10.

Die Modifikation von Proteinen, Polycarbonsäuren und anderen Biopolymeren durch Markieren mit einem Reportermolekül, welches insbesondere lumineszent sein kann, besitzt große Bedeutung für weite Anwendungsgebiete, wie zum Beispiel Immunologie, Zellbiologie oder auch für das Studium molekularer Wechselwirkungen, wie sie beispielsweise zwischen Liganden und Rezeptoren oder auch Proteinen und Nucleinsäuren stattfinden. Der Einsatz fluoreszenzmarkierter Moleküle ist nicht auf die Durchführung von Assays begrenzt. Hierbei gewinnt die Fluoreszenzmarkierung nicht nur in dem Maße an Bedeutung, wie radioaktive Assay-Methoden weniger zum Einsatz kommen. Die markierten Substanzen werden in fluoreszenzimmunologischen Assays (z. B. ELISA) oder in vielfältigen Fluoreszenztechniken, wie z. B. der Fluoreszenzmikroskopie, Fluoreszenzpolarisations- und Fluoreszenzlebenszeitmessungen oder der Fluoreszenzkorrelationsspektroskopie eingesetzt.

Verfahren zur Fluoreszenzmarkierung von Biopolymeren sind bereits bekannt. So ist es möglich Reaktivfarbstoffe, wie z. B. das Sulfonylchloridderivat von Sulforhodamin 101 (Texas Red®) durch direkte Zugabe zu einer Proteinlösung kovalent an Aminogruppen des Proteins zu koppeln. Hierbei ist jedoch die rasche Hydrolyse des Reaktivfarbstoffes in wäßriger Lösung bei Raumtemperatur nachteilig, da die Farbstoffmoleküle verfrüht inaktiviert werden, bevor diese in gewünschter Weise mit den zu markierenden Molekülen reagieren. Derartige Kopplungsreaktionen werden somit bevorzugt bei 4°C durchgeführt (Molecular Probes MP 353 12/21/93). Die Kopplungszeiten betragen je nach Wahl des Reaktivfarbstoffes und der Proteinkonzentration zwischen ein und vier Stunden (Titus et al., Journal of Immunological Methods, 50, 193 - 204, 1992). Rinderknecht (Experentia 16, 430, 1960) beschreibt eine weitere Arbeitsweise zur Fluoreszenzmarkierung unter Verwendung von auf Celite® adsorbierter Fluoreszenzfarbstoffe, welche der die zu markierenden Substanzen enthaltenden Lösung zugesetzt werden. Durch Bereitstellung der Fluoreszenzfarbstoffe auf einer derart vergrößerten Oberfläche lassen sich Kopplungszeiten von 30 Minuten erreichen. Nach der Reaktion wird das Celitematerial durch Zentrifugation abgetrennt.

Die oben genannten Verfahren besitzen insbesondere den Nachteil, daß die Durchführung der Verfahren eine im Umgang mit hydrolyseempfindlichen Substanzen erfahrene Person sowie eine zur Prophylaxe von Gesundheitsschäden gemäße Ausrüstung, wie zum Beispiel einen Abzug, erfordern. Als nachteilig hat sich zudem erwiesen, daß die Reproduzierbarkeit der Verfahren stark von subjektiven Umständen abhängig ist, wie Geschicklichkeit des die Reaktion ausführenden Personals.

Das der Erfindung zugrundeliegende technische Problem besteht in der industriellen Konfektionierung eines Verfahrens zur Markierung, insbesondere Fluoreszenzmarkierung, von Molekülen, welches reproduzierbar von nicht geschultem Personal oder automatisch durchgeführt werden kann.

Das der Erfindung zugrundeliegende Problem wird gelöst durch ein Verfahren zur Markierung von Molekülen gemäß Anspruch 1 und eine Vorrichtung zur Durchführung des erfindungsgemäßen Verfahrens nach Anspruch 10.

Das erfindungsgemäße Verfahren ist dadurch gekennzeichnet, daß die zu markierenden Moleküle in eine Reaktionskammer, in der eine mit einer Reaktivkomponente versehene Matrix angeordnet ist, gelangen, und die nach erfolgter Kopplung fluoreszenzmarkierten Moleküle die Reaktionskammer durch eine poröse Einrichtung verlassen.

Bei der Markierungsreaktion kann es sich in bevorzugter Weise um eine Fluoreszenzmarkierung handeln. Das erfindungsgemäße Verfahren eignet sich in vorteilhafter Weise auch zur Markierung mit vielfältigen anderen Reagenzien bzw. Substanzen, welche als solche detektierbar sind oder einen detektierbaren Anteil enthalten. So kann·erfindungsgemäß eine Markierung durch Haptene erfolgen, welche einer immunologischen Detektion zugänglich sind. Ebenso kann das erfindungsgemäße Verfahren auch zur Biotinylierung eingesetzt werden, so daß ein späterer Nachweis mittels Streptavidin bzw. Avidin erfolgen kann.

Der Austritt der markierten Moleküle aus der Reaktionskammer kann beispielsweise durch (hydrostatischen) Druck, Unterdruck oder Zentrifugation erfolgen.

Der Eintritt der zu markierenden Moleküle in die Reaktionskammer kann ebenso wie der Austritt der markierten Moleküle aus der Reaktionskammer durch eine poröse Einrichtung erfolgen.

Als zur Verwendung im erfindungsgemäßen Verfahren geeignete Matrix hat sich insbesondere eine Matrix aus anorganischen Materialien, wie Kieselgur verschiedener Korngrößen, zum Beispiel Celite®, erwiesen.

Bevorzugt handelt es sich bei den zu markierenden Molekülen um Substanzen, welche eine reaktive Amino-, Thiol- und/oder Aldehydgruppe aufweisen. Es können aber auch Carboxylgruppen nach Reaktion mit Carbodiimiden mit geeigneten Aminfarbstoffen markiert werden (M. Brinkley, Bioconjugate Chem., 3, 2 - 13, 1992). In besonders bevorzugter Weise sind die zu markierenden Substanzen natürliche oder synthetische Oligo- oder Polymere, wie Proteine, insbesondere Antikörper oder Enzyme, Nukleinsäuren, Kohlenhydrate, Polycarbonsäuren und Glykoproteine.

In bevorzugter Weise sind die Reaktivkomponenten aminoreaktive Reagenzien, wie fluoreszierende Isothiocyanatderivate, insbesondere Fluorescein-Isothiocyanat oder Tetramethylrhodamin-Isothiocyanat, Sulfonylchloridderivate, z. B. von Sulforhodamin 101 oder fluoreszierende Derivate von reaktiven Estern, wie z. B. N-Hydroxy-succinimidylester (NHS). Ebenso können erfindungsgemäß auch entsprechende Biotin- oder Haptenderivate verwendet werden. Erfindungsgemäß können ebenfalls thiolreaktive Reagenzien zur Anwendung gelangen, insbesondere fluoreszierende Derivate von Haloacetamiden oder Maleimidylderivate. Ferner können im erfindungsgemäßen Verfahren auch carboxyl- bzw. aldehydreaktive Reagenzien, wie Aminfarbstoffe oder Hydrazide, als Reaktivkomponenten verwendet werden (M. Brinkley, Bioconjugate Chem., 3, 2 - 13, 1992).

Die mittlere Porengröße der porösen Einrichtung sollte je nach Größe der zu markierenden Moleküle gewählt werden. Sie beträgt in bevorzugter Weise zwischen 50 µm und 100 µm, in besonders bevorzugter Weise zwischen 70 µm und 90 µm. Die Dicke der porösen Einrichtung sollte bevorzugt an das zu markierende System angepaßt werden. Als vorteilhaft haben sich Dicken < 2 mm erwiesen. Die poröse Einrichtung besteht insbesondere aus einem biokompatiblen, inerten Frittenmaterial, bevorzugt aus Polyethylen. In vorteilhafter Weise findet im Vergleich zum Stand der Technik durch den Austritt der markierten Moleküle aus der Reaktionskammer durch die poröse Einrichtung hindurch bereits eine Vorreinigung statt.

Die Temperatur zur Durchführung des erfindungsgemäßen Verfahrens sollte bevorzugt an das zu markierende System angepaßt werden. Geeignete Temperaturbereiche für die jeweilige Markierungsreaktion lassen sich durch Vorversuche ermitteln. So haben Parallelversuche - wie z. B. die Herstellung des Konjugates "Texas Red® - Alkalische Phosphatase" bei verschiedenen Temperaturen - ergeben, daß das Temperaturoptimum dieser Markierungsreaktion bei 24°C liegt. Bei empfindlicheren Substanzen kann es jedoch angebracht sein, das erfindungsgemäße Verfahren bei niedrigeren Temperaturen, insbesondere bei 4°C, durchzuführen.

Das erfindungsgemäße Verfahren eignet sich dazu, in einfacher Weise einen vorherbestimmten Markierungsgrad zu erzielen. Unter Markierungsgrad wird die Anzahl der kovalent gekoppelten Markierungsmoleküle pro markiertem Molekül verstanden.

Dies wird im folgenden Beispiel erläutert zur Herstellung von Konjugaten durch Reaktion von Resorufin-NHS mit Thyroglobulin. Es wurden in Parallelversuchen jeweils in einzelnen Reaktionskammern eine bestimmte Menge eines an eine Celite®-Matrix gekoppelten Resorufin-NHS vorgelegt und mit zunehmenden Mengen Thyroglobulin versetzt. So lassen sich erfindungsgemäß Markierungsgrade, in dem Fall gemäß Beispiel 1 zwischen 5 und 22, erzielen. Ebenso ließen sich unterschiedliche Markierungsgrade der alkalischen Phosphatase in Kopplungsreaktionen unter Verwendung von Texas Red®-Sulfonylchlorid, Fluorescein-Isothiocyanat (FITC), Tetramethylrhodamin-Isothiocyanat (TRITC), Succinimidylestern, wie BODIPY 581/591 (Molecular Probes), Iodacetamiden, wie z. B. Tetramethylrhodamin-Iodacetamid (TMR-IA), und Tetramethylrhodamin-Maleimid erzielen. Durch geeignete Wahl des Verhältnisses aus Reaktivkomponente und zu markierender Substanz ließen sich z. B. in der Kopplungsreaktion "Texas Red®-Lactalbumin" Markierungsgrade < 5 in gewünschter Weise beliebig einstellen. Auch ließen sich weitere Proteine wie Apoferritin und Lysozym in einfacher Weise reproduzierbar, z. B. unter Verwendung von Texas Red®-Sulfonylchlorid, markieren. Ebenso konnten Antikörper (Rabbit IgG) unter Verwendung von FITC, TRITC und den Succinimidylestern von Resorufin und Tetramethylrhodamin mit dem erfindungsgemäßen Verfahren markiert werden.

Das erfindungsgemäße Verfahren ist ebenso zur Markierung von Oligonukleotiden geeignet. In beispielhafter Weise wurde ein 40mer Oligonukleotid, welches am 5'-Ende mit NH₂ modifiziert war, unter Verwendung von Rhodamin Green®-NHS (Kopplungspuffer 0,1 M Na-Carbonatpuffer pH 10,5; 100-facher molarer Farbstoffüberschuß) markiert.

In einem weiteren Beispiel wurde das erfindungsgemäße Verfahren zur Biotinylierung von alkalischer Phosphatase unter Verwendung von Biotin-N-Hydroxysuccinimidylester als Reaktivkomponente eingesetzt.

Ein weiterer Vorteil des erfindungsgemäßen Verfahrens liegt in seiner schnellen Durchführbarkeit. Die bislang im Stand der Technik notwendigen Kopplungszeiten lassen sich unter Verwendung des erfindungsgemäßen Verfahrens deutlich auf wenige Sekunden verringern.

Messungen der Enzymaktivität z. B. von alkalischer Phosphatase zeigten, daß die unter Verwendung von Texas Red®-Sulfonylchlorid im erfindungsgemäßen Verfahren hergestellten Konjugate lediglich eine um wenige Prozent erniedrigte Aktivität im Vergleich zum nativen Enzym aufweisen.

Das erfindungsgemäße Verfahren ist mittels einer Vorrichtung gemäß Anspruch 9 durchführbar, welche einen im wesentlichen zylindrischen Hohlraum aufweist, in dessen Lumen mindestens eine poröse Einrichtung sowie eine mit einer Reaktivkomponente beladene Matrix angeordnet ist. In bevorzugter Weise befinden sich jedoch im Lumen zwei poröse Einrichtungen, zwischen denen die mit der Reaktivkomponente beladene Matrix angeordnet ist. Die Vorrichtung kann erfindungsgemäß insbesondere als eine aus einem inerten Material gefertigte Spritze mit unterschiedlicher Kapazität vorliegen. Vorzugsweise wird als biokompatibles, inertes Spritzenmaterial Polypropylen verwendet.

In einer bevorzugten Ausführungsform der erfindungsgemäßen Vorrichtung beträgt die Porengröße der porösen Einrichtung zwischen 50 µm und 100 µm, bevorzugt zwischen 70 µm und 90 µm.

In bevorzugter Weise wird eine aus anorganischen Materialien, wie Kieselgur verschiedener Korngröße, bestehende Matrix verwendet.

Die erfindungsgemäße Vorrichtung zeichnet sich durch ihre Lagerstabilität aus. Nach Beispiel 6 in geeigneter Weise bis zu 455 Tage lang gelagerte Spritzen zeigten keine signifikante Verschlechterung der Markierungsreaktion im Vergleich zu direkt nach dem Befüllen mit Texas Red®-Sulfonylchlorid-Celite® zur Markierung von alkalischer Phosphatase verwendeten Spritzen.

### Beispiel 1

### Herstellung einer Vorrichtung zur Durchführung des erfindungsgemäßen Verfahrens

Der Kolben einer aus Polypropylen gefertigten Spritze wurde aus dieser entnommen und der Auslauf der Spritze mit einer aus Polyethylen bestehenden porösen Einrichtung versehen, deren Durchmesser an den Innendurchmesser des Spritzenhohlraumes angepaßt war. Die Dicke der porösen Einrichtung betrug 2 mm, die mittlere Porengröße 80 µm. Zusammen mit einer weiteren entsprechenden porösen Einrichtung wurden der Kolben und die zugehörige Spritze bis ca. eine halbe Stunde vor Gebrauch bei 50°C getrocknet. 20 mg Celite® wurden in ein Rotationsverdampferspitzkölbchen eingewogen. Das mit Celite® versehene Spitzkölbchen wurde zusammen mit der porösen Einrichtung, dem Kolben und der vorbereiteten Spritze in einen Exsikkator gestellt, welcher anschließend evakuiert wurde. Danach wurde der Exsikkator mit Argon geflutet und bei halb geöffnetem Deckel mit einem Schlauch, der bis auf den Boden des Exsikkators reichte, ständig mit Argon nachbefüllt. Eine gewünschte Menge z. B. 0,1 mg Texas Red®-Sulfonylchlorid wurde im Exsikkator sukzessiv in Chloroform gelöst und anschließend vollständig auf das Celite® überführt. Der Exsikkator wurde verschlossen und für ca. eine halbe Stunde Vakuum angelegt. Vor der Weiterverarbeitung wurde die mit Texas Red®-Sufonylchlorid versehen Celite®-Matrix für weitere zwei Stunden im Exsikkator belassen. Das Befüllen der Spritze erfolgte im Exsikkator. Hierzu wurde dieser wiederum mit Argon geflutet und bei halb geöffnetem Deckel mit einem bis zum Exsikkatorboden reichenden Schlauch ständig mit Argon nachbefüllt. Die mit der Reaktivkomponente Texas Red®-Sulfonylchlorid versehene Celite®-Matrix wurde in die Spritze eingefüllt. Eine zweite poröse Einrichtung wurde anschließend in die Spritze eingesetzt. Bei Einsatz des Spritzenkolbens wurde darauf geachtet, daß die sich somit zwischen zwei porösen Einrichtungen befindende, mit Texas Red®-Sulfonylchlorid versehene Celite®-Matrix nur in geringem Maße verdichtet wurde. Es ist jedoch ebenso möglich, auf die zweite poröse Einrichtung, die den Raum des Farbstoff-Celite®-Komplexes in der Spritze begrenzt, zu verzichten. Nach Einsetzen des Spritzenkolbens kann mit dem Kolbenhub der Reaktionsraum innerhalb der Spritze variabel gehalten werden.

### Beispiel 2

### Markierung von Thyroglobulin mit verschiedenen Markierungsgraden

Es wurden in Parallelversuchen jeweils in einzelnen Reaktionskammern 210 nmol an eine Celite®-Matrix gekoppeltes Resorufin-NHS vorgelegt und mit je 100 µl Kopplungspuffer (0,1 M Na-Boratpuffer, pH 9,0) enthaltend a) 7,1 mg Thyroglobulin (entspricht 20-fachem molaren Farbstoffüberschuß), b) 3,6 mg Thyroglobulin (entspricht 40-fachem molaren Farbstoffüberschuß), c) 1,42 mg Thyroglobulin (entspricht 100-fachem molaren Farbstoffüberschuß, d) 0,71 mg Thyroglobulin (entspricht 200-fachem molaren Farbstoffüberschuß) und e) 0,36 mg Thyroglobulin (entspricht 400-fachem molaren Farbstoffüberschuß) versetzt. Die Reaktionskammern wurden viermal mit jeweils 100 µl Kopplungspuffer nachgespült und das Eluat jeweils zum ersten Eluat gegeben. Das gesamt Eluat wurde in an sich bekannter Weise chromatographisch gereinigt. Die Konzentrationen des Biomoleküls und des Farbstoffes der Konjugatlösung wurden in bekannter Weise spektroskopisch über die molaren Extinktionskoeffizienten bestimmt.

Es wurden die folgenden Markierungsgrade erzielt:

| molarer Farbstoffüberschuß während der Reaktion | Markierungsgrad |
|---|---|
| 20 | 6,2 |
| 40 | 8,8 |
| 100 | 20 |
| 200 | 22 |
| 400 | 22 |

### Beispiel 3

### Markierung von alkalischer Phosphatase mit Texas Red®-Sufonylchlorid

50 µl der Enzymlösung (10 mg alkalische Phosphatase / ml Kopplungspuffer) wurden in einem 1,5 ml fassenden Eppendorf®-Gefäß vorgelegt und mit 50 µl Kopplungspuffer (0,1 M Na-Boratpuffer, pH 9,0) gemischt, welcher auf 24°C vortemperiert war. Eine Injektionskanüle wurde auf die Vorrichtung, hier eine Spritze, aufgesetzt und damit die Enzymlösung in die Vorrichtung aufgezogen, kurz durch Kolbenbewegung gemischt und das Eluat in ein auf Eis befindliches Eppendorf®-Gefäß gegeben. Die Vorrichtung wurde anschließend viermal mit 100 µl Kopplungspuffer gespült und das Eluat jeweils in das Eppendorf®-Gefäß zugegeben. Das gesamte Eluat wurde in bekannter Weise chromatographisch gereinigt.

### Beispiel 4

### Markierung von Antikörpern

Unter Verwendung von mit FITC sowie den Succinimidylestern von Texas Red®, Resorufin und Tetramethylrhodamin (jeweils ca. 200 nmol Farbstoff pro Vorrichtung) bestückten Celite®-Matrices wurde Rabbit IgG in der erfindungsgemäßen Vorrichtung markiert. Zunächst wurden 2,8 mg Antikörper in 200 µl 0,1 M Na-Boratpuffer (pH 9,0) gelöst. 30 µl dieser Lösung wurden sodann mit 70 µl 0,1 M Na-Boratpuffer gemischt. Eine Injektionskanüle wurde auf die Vorrichtung, hier eine Spritze, aufgesetzt und damit die Antikörperlösung in die Vorrichtung aufgezogen, kurz durch Kolbenbewegung gemischt und das Eluat in ein auf Eis befindliches Eppendorf®-Gefäß gegeben. Die Vorrichtung wurde anschließend viermal mit 100 µl Kopplungspuffer gespült und das Eluat jeweils in das Eppendorf®-Gefäß zugegeben. Das gesamte Eluat wurde in bekannter Weise chromatographisch gereinigt. Es wurden Markierungsgrade von 7,0 (FITC), 7,7 (Resorufin-NHS) , 1,8 (Tetramethylrhodamin-Succinimidylester) und 0,7 (TRITC) erzielt.

### Beispiel 5

### Biotinylierung von alkalischer Phosphatase

100 µl der Enzymlösung (10 mg alkalische Phosphatase / ml 0,1 M Na-Boratpuffer pH 9,0) wurden unter Verwendung von Biotin-NHS als Reaktivkomponente in Anlehnung an Beispiel 3 markiert.

### Beispiel 6

### Lagerstabilität

Mit Texas Red®Sufonylchlorid-Celite® befüllte Spritzen wurden in Flachsäcken Barr-O-Nit® mittels eines Impulsschweißgerätes einzeln verpackt und bei -20°C gelagert. Nach 7, 13, 21, 27, 41, 76 und 455 Tagen Lagerung wurden Spritzen zur Markierung von alkalischer Phosphatase verwendet. Die Markierungsgrade wurden bestimmt und mit dem erzielten Markierungsgrad einer direkt nach dem Befüllen mit Texas Red®-Sulfonylchlorid-Celite® zur Markierung von alkalischer Phosphatase verwendeten Spritze verglichen.

| Lagerdauer (Tage) | Markierungsgrad |
|---|---|
| 0 | 2,3 |
| 7 | 2,2 |
| 13 | 1,9 |
| 21 | 2,2 |
| 27 | 2,1 |
| 41 | 2,2 |
| 76 | 2,1 |
| 455 | 2,3 |

## Patentansprüche

1. Verfahren zur Markierung von Molekülen durch Reaktion von zu markierenden Molekülen mit Markern an einer festen Phase (Matrix), **dadurch gekennzeichnet, daß** die Moleküle in eine Reaktionskammer, in der eine mit einer Reaktivkomponente versehene Matrix angeordnet ist, gelangen, und die nach erfolgter Kopplung markierten Moleküle die Reaktionskammer durch eine poröse Einrichtung verlassen.

2. Verfahren gemäß Anspruch 1, wobei die Markierung eine Fluoreszenzmarkierung, Biotinylierung und/oder Haptenylierung ist.

3. Verfahren gemäß Anspruch 1 und/oder 2, wobei der Austritt der fluoreszenzmarkierten Moleküle aus der Reaktionskammer durch (Unter-)Druck oder Zentrifugation erfolgt.

4. Verfahren gemäß mindestens einem der Ansprüche 1 bis 3, wobei der Eintritt der zu markierenden Moleküle in die Reaktionskammer durch eine poröse Einrichtung erfolgt.

5. Verfahren gemäß mindestens einem der Ansprüche 1 bis 4, wobei als Matrix anorganische Materialien, wie Kieselgur verschiedener Korngrößen verwendet werden.

6. Verfahren gemäß mindestens einem der Ansprüche 1 bis 5, wobei die zu markierenden Moleküle mindestens eine nukleophile oder elektrophile Gruppe, wie Amino-, Thiol- und/oder Aldehydgruppe, aufweisen.

7. Verfahren gemäß mindestens einem der Ansprüche 1 bis 6, wobei die zu markierenden Moleküle natürliche oder synthetische Oligo- oder Polymere, wie Proteine, insbesondere Antikörper oder Enzyme, Nukleinsäuren, Kohlenhydrate, Polycarbonsäuren und Glykoproteine sind.

8. Verfahren gemäß mindestens einem der Ansprüche 1 bis 7, wobei die Reaktivkomponenten ein fluoreszierendes Derivat, ein Haptenderivat oder Biotinderivat von Isothiocyanaten, Sulfonylchloriden, N-Hydroxysuccinimidylestern, Haloacetamiden oder Maleimiden ist.

9. Verfahren gemäß mindestens einem der Ansprüche 1 bis 8, wobei die Porengröße der porösen Einrichtung zwischen 50 µm und 100 µm, bevorzugt zwischen 70 µm und 90 µm, beträgt.

10. Vorrichtung zur Durchführung des Verfahrens nach mindestens einem der Ansprüche 1 bis 9 mit einem Hohlraum, in dessen Lumen mindestens eine poröse Einrichtung sowie eine mit einer Reaktivkomponente beladene Matrix angeordnet ist.

## Claims

1. A process for the labeling of molecules by the reaction of molecules to be labeled with markers on a solid phase (matrix), **characterized in that** said molecules are passed into a reaction chamber which contains a matrix provided with a reactive component, and after coupling has been effected, the labeled molecules leave the reaction chamber through a porous device.

2. The process according to claim 1, wherein said labeling is fluorescent labeling, biotinylation and/or haptenylation.

3. The process according to claim 1 and/or 2, wherein the exit of the fluorescent-labeled molecules from the reaction chamber is effected by (reduced) pressure or centrifugation.

4. The process according to at least one of claims 1 to 3, wherein said passing of the molecules to be labeled into the reaction chamber is effected through a porous device.

5. The process according to at least one of claims 1 to 4, wherein inorganic materials, such as kieselguhr of different grain sizes, are used as said matrix.

6. The process according to at least one of claims 1 to 5, wherein the molecules to be labeled have at least one nucleophilic or electrophilic group, such as amino, thiol and/or aldehyde group.

7. The process according to at least one of claims 1 to 6, wherein the molecules to be labeled are natural or synthetic oligomers or polymers, such as proteins, especially antibodies or enzymes, nucleic acids, carbohydrates, polycarboxylic acids and glycoproteins.

8. The process according to at least one of claims 1 to 7, wherein said reactive components are a fluorescent derivative, a haptene derivative or biotin derivative of isothiocyanates, sulfonyl chlorides, N-hydroxysuccinimidyl esters, haloacetamides or maleimides.

9. The process according to at least one of claims 1 to 8, wherein the pore size of said porous device is between 50 µm and 100 µm, preferably between 70 µm and 90 µm.

10. A device for performing the process according to at least one of claims 1 to 9, comprising a cavity in the lumen of which at least one porous device and at least one matrix loaded with a reactive component are provided.

## Revendications

1. Procédé de marquage de molécules par réaction de molécules à marquer avec des marqueurs sur une phase solide (matrice), **caractérisé en ce que** les molécules migrent dans une chambre de réaction dans laquelle une matrice dotée d'un composant réactif est disposée, et **en ce que** les molécules marquées, une fois le couplage effectué, sortent de la chambre de réaction par un dispositif poreux.

2. Procédé selon la revendication 1, dans lequel le marquage est un marquage par fluorescence, une biotinylation et ou une haptènisation.

3. Procédé selon la revendication 1 et/ou 2, dans lequel la sortie des molécules marquées par fluorescence hors de la chambre de réaction s'effectue par pression (dépression) ou par centrifugation.

4. Procédé selon au moins une des revendications 1 à 3, dans lequel l'entrée des molécules à marquer dans la chambre de réaction s'effectue en passant par un dispositif poreux.

5. Procédé selon au moins une des revendications 1 à 4, **caractérisé en ce qu'**en tant que matrice, l'on utilise des matériaux minéraux tels que du gel de silice de différentes granulométries.

6. Procédé selon au moins une des revendications 1 à 5, dans lequel les molécules à marquer comportent au moins un groupe nucléophile ou électrophile tel qu'un groupe amine, un groupe thiol et/ou un groupe aldéhyde.

7. Procédé selon au moins une des revendications 1 à 6, dans lequel les molécules à marquer sont des oligomères ou des polymères naturels ou synthétiques, tels que des protéines, notamment des anticorps ou des enzymes, des acides nucléiques, des hydrates de carbone, des acides polycarboxyliques et des glycoprotéines.

8. Procédé selon au moins une des revendications 1 à 7, dans lequel le composant réactif est un dérivé fluorescent, un dérivé haptènique ou un dérivé biotinique d'isothiocyanates, de chlorures de sulfonyle, de N-hydroxysuccinimidylesters, d'haloacétamides ou de maléimides.

9. Procédé selon au moins une des revendications 1 à 8, dans lequel la taille des pores du dispositif poreux est de 50 µm à 100 µm, de préférence de 70 µm à 90 µm.

10. Montage pour réaliser le procédé selon au moins une des revendications 1 à 9 comportant un creux dans le parcours lumineux duquel est disposé au moins un dispositif poreux ainsi qu'une matrice chargée avec un composant réactif.
